# EUROPEAN PATENT APPLICATION

(11) **EP 4 023 658 A1**
(43) Date of publication of application: **06.07.2022**
(21) Application number: 20217894.3
(22) Date of filing: 30.12.2020
(51) Int. Cl.: C07H 1/00, C07H 21/00, C07H 21/02

(54) **MODIFIED NUCLEIC ACID CONJUGATES**

(71) Applicant: Fuchs, Merle, 07552 Gera (DE); Technische Universität Berlin, 10623 Berlin (DE)
(72) Inventor: The designation of the inventor has not yet been filed
(74) Representative: Weiss, Wolfgang

(57) **Abstract**

The present invention refers to a nucleic acid conjugate comprising at least one 2',3'-O ketal moiety. This conjugate is suitable for the target-specific or target-selective delivery of nucleic acid molecules into cells such as mammalian cells including human cells with high efficacy. Thus, a new delivery vehicle for therapeutic nucleic acid molecules including antisense molecules, siRNA molecules, miRNA molecules, antagomirs or precursors of such molecules is provided.

SEQUENCE LISTING

<110>  Merle Fuchs; Technische Universität Berlin

<120>  Modified nucleic acid conjugates

<130>  70570P EP

<160>  4

<170>  PatentIn version 3.5

<210>  1
<211>  21
<212>  RNA
<213>  artificial sequence

<220>
<223>  GAPDH-antisense

<400>  1

aaaguuguca uggaugacct t

<210>  2
<211>  21
<212>  RNA
<213>  artificial sequence

<220>
<223>  GAPDH-sense

<400>  2

ggucauccau gacaacuuut t

<210>  3
<211>  21
<212>  RNA
<213>  artificial sequence

<220>
<223>  Scr-antisense

<400>  3

gaacgaauuu auaaguggct t

<210>  4
<211>  21
<212>  RNA
<213>  artificial sequence

<220>
<223>  Scr-sense

<400>  4

gccacuuaua uaaauucguuct t

## Description

The present invention refers to a nucleic acid conjugate comprising at least one 2',3'-O ketal moiety. This conjugate is suitable for the target-specific or target-selective delivery of nucleic acid molecules into cells such as mammalian cells, including human cells with high efficacy. Thus, a new delivery vehicle for therapeutic nucleic acid molecules including antisense molecules, and siRNA molecules or precursors or modifications of such molecules is provided.

WO 2014/048969 describes nucleolipids and a process for the preparation of nucleolipids. *Inter alia,* nucleotides comprising a lipid 2',3'-O ketal moiety are described. These nucleotides were found to have pharmaceutical activity in certain cancer cells. The delivery of nucleic acid molecules comprising 2',3'-O-ketal moieties into target cells is, however, not disclosed.

It is an object of the present invention to provide nucleic acid molecules, which can be delivered into target cells with high efficacy.

The inventors have found that attaching a 2',3'-O ketal moiety to a nucleic acid molecule, particularly to the 3'-end and/or to the 5'-end of a nucleic acid strand, may increase the delivery into a target cell, particularly the *in vivo* delivery into a target cell or target organism. Thus, therapeutic or diagnostic nucleic acid conjugates with improved characteristics are provided.

A first aspect of the present invention is a nucleic acid conjugate of the formula (I) comprising at least one 2',3'-O-ketal moiety: 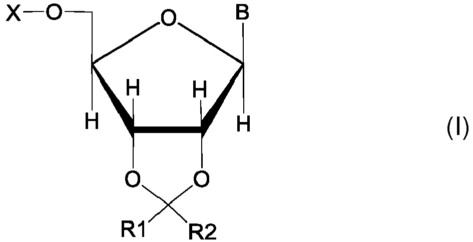 wherein
X is a nucleic acid molecule,
B is a nucleobase, and
R1 and R2 are independently from each other hydrocarbon groups wherein said hydrocarbon groups optionally comprises one or more heteroatom(s) such as N, O, P, S, or halo.

It is understood that the invention also includes any stereoisomers, salts and solvates of the compound depicted in formula (I) and any other formula herein.

The nucleic acid conjugate of the formula (I) comprises a nucleic acid molecule X, to which at least one 2',3'-O-ketal moiety is attached.

The term *"nucleic acid molecule"* relates to any type of oligonucleotide or polynucleotide comprising at least two nucleotide building blocks. The nucleic acid molecule may comprise ribonucleotide building blocks, 2'-desoxyribonucleotide building blocks, modified nucleotide building blocks or any combinations thereof. In certain embodiments, the nucleic acid molecule is an RNA molecule, optionally comprising at least one modified nucleotide building block and/or at least one 2'-desoxyribonucleotide building block, a DNA molecule, optionally comprising at least one modified nucleotide building block and/or at least one ribonucleotide building block, or a nucleic acid analogue molecule consisting of modified nucleotide building blocks.

The 2',3'-O ketal moiety comprises a nucleobase B. The term *"nucleobase"* typically relates to a cyclic, e.g. mono- or bicyclic, saturated, unsaturated, aromatic or heteroaromatic base comprising at least one N atom wherein the nucleobase is capable of forming a base pair via hydrogen bonds with a complementary nucleobase, particularly with a naturally occurring nucleobase. The nucleobase may be any naturally occurring or non-naturally occurring nucleobase, e.g. any naturally occurring or non-naturally occurring purine or pyrimidine base such as adenine, cytosine, guanine, thymine, or uracil or a modified nucleobase, e.g. a modified adenine, cytosine, guanine, thymine, or uracil base.

The nucleobase of the 2',3'-O ketal moiety may be a *"modified nucleobase".* This term includes any type of modified nucleobase, e.g. a nucleobase substituted with a hydrophobic moiety, a nucleobase substituted with a carbohydrate moiety and/or a nucleobase substituted with a functional moiety.

The hydrophobic moiety may be selected from moieties having at least 5 C-atoms, particularly at least 10 C-atoms.

In certain embodiments, the hydrophobic moiety is selected from substituted or unsubstituted acyclic or cyclic terpene moieties. For example, the hydrophobic moiety may be a C₅-C₃₀ terpene moiety, particularly a C₅-C₂₀ terpene moiety, and more particularly a C₁₅ terpene moiety, which may be cyclic or acyclic, which may be saturated or unsaturated, and/or optionally be substituted or interrupted with heteroatom(s) or functional group(s), or a sterol moiety, e.g. a cholesterol moiety.

Further, the hydrophobic moiety may be selected from substituted or unsubstituted, cyclic or acyclic, saturated, unsaturated, or polyunsaturated carboxylic acid moieties including salts or derivatives thereof such as esters or amides. For example, the hydrophobic moiety may be a C₅-C₃₀ carboxylic acid moiety, e.g. a docosahexaenoic acid moiety, an eicosapentaenoic acid moiety, a docosanoic acid moiety, a lithocholic acid moiety, or a retinoic acid or retinoic acid ester moiety.

Further, the hydrophobic moiety may be selected from substituted or unsubstituted, cyclic, or acyclic, saturated, unsaturated or polyunsaturated alcohols, ketones, aldehydes, or amines. For example, the hydrophobic moiety may be a C₅-C₃₀ alcohol, ketone, or amide moiety, e.g. a tocopherol moiety, a tocopheryl succinate moiety, a retinol moiety, a retinal moiety, a spermine moiety, or a spermidine moiety.

In certain embodiments, the hydrophobic moiety is selected from 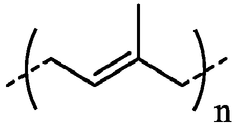 and/or 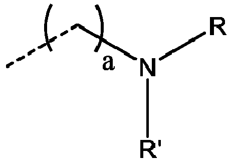 wherein
R and R' are independently selected from C₁-C₃₀ alkyl, preferably C₅ to C₂₅-alkyl, n is an integer ranging 1 to 6, preferably n is 1 or 2 and a is an integer ranging from 1 to 20, preferably 2 to 18, more preferably 6 to 16.

The carbohydrate moiety may be a mono-, oligo- or polysaccharide moiety, including modified carbohydrates, e.g. acetylated carbohydrate moieties. For example, the carbohydrate moiety may be a GalNAc moiety.

The functional moiety may be a click-functional moiety such as an alkyne group or an azide group, or an ether, ester, amide, carboxylic acid, thioester, thioamide, or thioether group.

The conjugate of formula (I) comprises a 2',3'-O-ketal moiety wherein the 2'-OH group and the 3'-OH group of the ribose moiety have been converted to a ketal group comprising substituents R1 and R2.

R1 and R2 are independently from each other hydrocarbon groups wherein said hydrocarbon groups optionally comprise one or more heteroatom(s) such as N, O, P, S, or halo.

R1 and R2 may be saturated, mono- or polyunsaturated acyclic linear or branched hydrocarbon groups, or saturated, mono- or polyunsaturated, aromatic or heteroaromatic cyclic hydrocarbon groups, which may be interrupted by or substituted with one or more heteroatom(s).

In certain embodiments, R1 and/or R2 are independently from each other C₅-₂₁ hydrocarbon groups, particularly C₅₋₁₁ hydrocarbon groups and more particularly C₉ hydrocarbon groups wherein said hydrocarbon groups optionally comprise one or more heteroatoms such as N, O, P, S, or halo, e.g. F, Cl, Br, or I.

In further embodiments, R1 and/or R2 are independently from each other linear or branched alkyl groups, linear or branched alkenyl groups, and linear or branched alkynyl groups wherein said alkyl, alkenyl or alkynyl groups optionally comprise one or more heteroatoms such as N, O, P, S, or halo.

In still further embodiments, R1 and/or R2 are independently from each other cyclic moieties, i.e. moieties comprising at least one cyclic structure, e.g. a mono-, bi- or tricyclic structure. The cyclic moiety may be a carbocyclic or heterocyclic moiety, e.g. 5-18 ring atoms wherein at least one C-atom may be replaced by at least one heteroatom selected from N, O, and S.

In still further embodiments, R1 and/or R2 comprise independently from each other, a functional group, e.g. a functional group selected from an ether, ester, amide, carboxylic acid, thioester, thioamide, or thioether group.

In still further embodiments, R1 and/or R2 comprise independently from each other, a marker group, e.g. a fluorescence, luminescence, or radioactive marker group.

In still further embodiments, R1 and/or R2 comprise independently from each other, a nucleic acid molecule as herein defined, e.g. a nucleic molecule having up to 50 and particularly from 5 to 25 nucleotide building blocks.

In particular embodiments, R1 and R2 are linear or branched C₉ alkyl groups, more particularly linear C₉ alkyl groups.

In a specific embodiment, the conjugate comprises a nucleobase B, which is an uracil base of the formula (IIa): 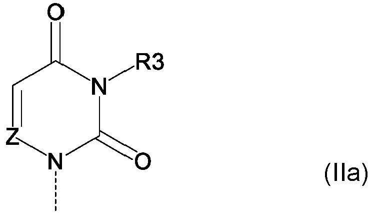 wherein Z is CH or N and R3 is H.

In this embodiment, R1 and R2 are preferably linear C₉ alkyl groups.

In a further specific embodiment, the conjugate comprises a nucleobase B, which is an uracil base of the formula (IIb): 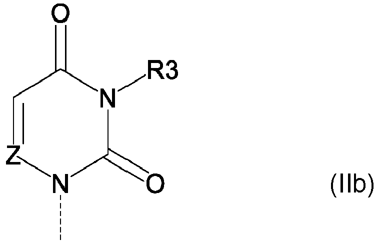 wherein Z is CH or N and R3 is a hydrophobic moiety, e.g. terpene moiety, particularly a C₅-C₃₀ terpene moiety, more particularly a C₅-C₂₀ terpene moiety, and even more particularly a C₁₅ terpene moiety, e.g. a terpene moiety of the formula (III):

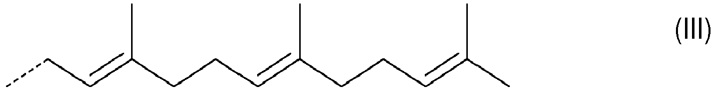

In this embodiment, R1 and R2 are preferably linear C₉ alkyl groups.

In certain embodiments, the nucleic acid molecule X has a strand length of at least about 5 nucleotide building blocks and up to 1,000 or more nucleotide building blocks. In further embodiments, the nucleic acid molecule X has a strand length between about 5 to about 100 nucleotide building blocks, between about 10 and about 50 nucleotide building blocks, between about 12 and about 40 nucleotide building blocks and particularly between about 15 and about 30 nucleotide building blocks.

The term *"nucleic acid molecule"* encompasses single-stranded, double-stranded, triple-stranded, and quadruple-stranded nucleic acid molecules, e.g. DNA molecules or RNA molecules and analogues thereof. Further, the nucleic acid molecule may comprises at least one modified nucleotide building block as described below.

In one embodiment, the nucleic acid molecule is a DNA molecule, which may comprise at least one modified nucleotide building block and/or at least one ribonucleoside building block. The term *"DNA molecule"* encompasses single-stranded, and multi-stranded, e.g. double-stranded, triple-stranded, or quadruple-stranded DNA molecules, particularly single- or double stranded DNA molecules. Multi-stranded, e.g. double-stranded DNA molecules may comprise strands having the same length or strands having different lengths. In multi-stranded, e.g. double-stranded DNA molecules, the individual strands may be present as separate molecules or covalently connected via a single-stranded loop or via heterologous linker.

The term *"DNA molecule"* encompasses molecules consisting of natural DNA building blocks, i.e. 2'-deoxyribonucleotide building blocks, and molecules comprising at least one 2'-deoxyribonucleotide building block and at least one modified nucleotide building block and/or at least one ribonucleotide building block.

In a further embodiment, the nucleic acid molecule is an RNA molecule, which may comprise at least one modified building block and/or at least one 2'-deoxyribonucleoside building block. The term *"RNA molecule"* encompasses single-stranded, or multi-stranded, e.g. double-stranded, triple-stranded, and quadruple-stranded RNA molecules, particularly single- or double stranded RNA molecules. Multi-stranded RNA molecules may comprise strands having the same length or strands having different lengths. For example, double-stranded RNA molecules may be blunt-ended or may have at least one overhang, e.g. at least one 3'-overhang. In multi-stranded, e.g. double-stranded RNA molecules, the individual strands may be present as separate molecules or covalently connected via a single-stranded loop or via a heterologous linker.

The term *"RNA molecule"* encompasses molecules consisting of natural RNA building blocks, i.e. ribonucleotide building blocks, and molecules comprising natural RNA building blocks and at least one modified nucleotide building block and/or at least one 2'-deoxyribonucleoside building block.

The term *"nucleotide building block"* relates to a moiety, which can be incorporated into a nucleic acid molecule as herein described and form part of said nucleic acid molecule. Typically, a nucleotide building block comprises a nucleobase moiety, a sugar moiety and a backbone moiety. The term *"nucleobase"* may encompass any type of nucleobase as herein described above for the nucleobase B of formula (I). The sugar moiety may encompass a ribose moiety or a 2'-deoxyribose moiety including any modification thereof. The backbone moiety may encompass a phosphoester group forming an internucleosidic linkage between two nucleotide building blocks including any modification thereof. The nucleic acid molecule X may comprise modified nucleotide analogue blocks comprising a nucleobase modification, a sugar modification and/or a backbone modification.

In certain embodiments, the nucleic acid molecule X comprises at least one modified nucleotide building block, particularly selected from:
(a) a nucleobase-modified building block;
(b) a sugar-modified building block;
(c) a backbone-modified building block and
(d) any combination thereof.

The term *"nucleobase-modified building block"* relates to a nucleotide building comprising a modified nucleobase. The term *"modified nucleobase"* may encompass any type of modified nucleobase as herein described above for the nucleobase B of formula (I).

The term *"sugar-modified building block"* "relates to a nucleotide building comprising a modified sugar. The term *"sugar"* typically relates to a ribose or 2'-deoxyribose moiety and includes any type of modified sugar moiety. In certain embodiments, a sugar-modified nucleotide building block is selected from a nucleotide building block wherein the ring of the ribose moiety and/or the substituents on the ring are modified.

In certain embodiments, the modified nucleotide building block is a 2'-modified nucleotide building block wherein the 2'-OH substituent of ribose moiety is replaced by 2'-R4 wherein R4 is halo, e.g., fluoro, C₁-C₅ alkyl, O-C₁-C₅ alkyl, S- C₁-C₅ alkyl, C₂-C₅ alkenyl, O-C₂-C₅ alkenyl, S-C₂-C₅ alkenyl, C₂-C₅ alkynyl, O-C₂-C₅ alkynyl, S-C₂-C₅ alkynyl, amino including mono- or disubstituted amino, e.g., C₁-C₅ alkyl, C₂-C₅ alkenyl or C₂-C₅ alkynyl substituted amino wherein each alkyl, alkenyl or alkynyl group is optionally substituted with OH, halo, cycloalkyl, cycloalkenyl, (hetero)aryl, e.g. phenyl, O-alkyl, S-alkyl, and/or amino.

In certain embodiments, the modified nucleotide building block is a bridged, e.g. a 2'-4'-bridged modified nucleotide building block, wherein the bridge typically has a length of 2-5 atoms, particularly 2-3 atoms including C-atoms and optionally heteroatoms such as O, N or S. In a particular embodiment, the modified building block is a locked nucleic building block having a 2'-O-CH₂-4' bridge.

In certain embodiments, the modified nucleotide building block is building block wherein the ring of the ribose moiety is modified, e.g. a morpholino building block, a thio-ribose building block, a 6-membered pyranose building block or a peptidic nucleic acid (PNA) building block.

The term *"backbone-modified building block"* relates to a nucleotide building comprising a modified internucleosidic linkage. In a backbone-modified nucleotide building block, the phosphoester group connecting adjacent building blocks is replaced by modified internucleosidic linkage, e.g. a phosphorothioate linkage, an alkyl phosphonate, e.g. methyl phosphonate linkage, and a borano phosphate linkage.

The nucleic acid molecule is preferably selected from nucleic acid molecules, which are suitable for pharmaceutical applications. Preferred nucleic acid molecules include mRNA molecules comprising coding sequences, particularly mRNA molecules comprising protein-coding sequences, RNA molecules capable of RNA interference such as 16-27-mer siRNAs, particularly 21-mer siRNAs, blunt siRNAs, sisiRNAs, shRNAs, asiRNAs, aiRNAs, Fork siRNAs, 27-mer siRNA, Dumbbell siRNA, 16 mer-siRNAs, ss-siRNAs; microRNAmolecules and antagomirs thereof, e.g. pre-miRNAmimics; RNA molecules capable of gene-editing, e.g. constituents of the CRISPR/Cas complex such as tracrRNAs, crRNAs, sgRNAs; antisense DNAs or RNAs; triplex or quadruplex forming nucleic acid molecules; CpG-oligonucleotides, TTAGGG-oligonucleotides, aptamers, ribozymes, or DNAzymes or precursors or modifications of such molecules

In certain embodiments, the nucleic acid molecule X is an RNA molecule optionally comprising at least one modified building block and/or at least one DNA building block, a DNA molecule optionally comprising at least one modified building block and/or at least one RNA building block, or a nucleic acid analogue molecule consisting of modified building blocks.

In further embodiments, the nucleic acid molecule X is a double-stranded RNA molecule optionally comprising at least one modified building block and optionally having at least one overhang, e.g., a 5'-overhang and/or a 3'-overhang, e.g. an overhang of 1, 2 or 3 nucleotide building blocks. In particular embodiments, the RNA molecule is a siRNA molecule.

In further embodiments, the nucleic acid molecule X is a single-stranded DNA molecule optionally comprising at least one modified building block and/or at least one ribose building block, particularly an antisense molecule.

The nucleic acid conjugate of the formula (I) may be prepared by attaching a 2'-3'-O-ketal moiety (IV) to the nucleic acid molecule X, wherein the 2'-3'-O-ketal moiety is of the formula (IV): 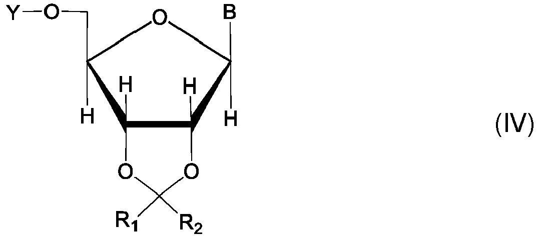 wherein
Y is a reactive functional moiety, e.g. a phosphoramidate group or another group suitable in the chemical synthesis nucleic acid molecules,
B is a nucleobase, and
R1 and R2 are independently from each other hydrocarbon groups wherein said hydrocarbon groups optionally comprise one or more heteroatoms such as N, O, P, S, or halo.

The synthesis of the 2',3'-O-ketal moiety (IV) is described in WO 2014/048969, the disclosure of which is herein incorporated by reference. The attachment of the 2',3'-O-ketal moiety (IV) to the nucleic acid molecule X may be performed during or after synthesis of the nucleic acid molecule X, e.g. according to standard methods in solid phase nucleic acid synthesis.

In certain embodiments, a 2',3'-O-ketal building block is attached to the 5'-terminus and/or to the 3'-terminus of at least one strand of the nucleic acid molecule X.

In specific embodiments, a 2',3'-O-ketal building block is attached to the 5'-terminus of the sense strand of a double-stranded siRNA molecule or to the 5'-terminus of an antisense molecule.

In further embodiments, a 2',3'-O-ketal building block is attached to a nucleobase within a strand of the nucleic acid molecule X.

In further embodiments, there is a plurality, e.g. 2 of 2',3'-O-ketal building blocks attached to the nucleic acid molecule X.

A further aspect of the invention relates to the use of the conjugate of the formula (I) in medicine, e.g., for use in human medicine or veterinary medicine, particularly for use in human medicine and *in vivo* animal research. In certain embodiments, the medical use comprises administering the conjugate to a subject in need thereof, particularly to a human subject. In further embodiments, the conjugate is administered to a target cell or a target organ *ex vivo* and the target cell or target organ is subsequently introduced into a subject in need thereof, particularly into a human subject. In still further embodiments, the conjugate is administered to an oocyte or embryo, wherein the use of human stem cells or human embryos for industrial commercial purposes is excluded.

The present invention provides an efficient method of mediating target-specific nucleic acid modifications in a cell or an organism comprising the steps:
(a) contacting a cell or organism with the conjugate of the invention, and
(b) mediating a target-specific nucleic acid modification effected by the nucleosidic component of the conjugate towards a target nucleic acid.

Contacting step (a) may comprise introducing the conjugate into a target cell, e.g., an isolated target cell, which may be present in a cell culture, a unicellular micro-organism, or a target cell, or a plurality of target cells within a multicellular organism. The target cell is preferably a mammalian cell, including a human cell. The target organism is preferably a mammalian organism, e.g. a human organism.

The introducing into an organism may comprise any type of administration including systemic or local administration, e.g. by injection or infusion. Exemplary types of administration include aural, buccal, endobronchial, enteral, epidural, inhalative, instillation into the bladder, intra-arterial, intra-articular, intragastric, intragluteal, intracardiac, intracutaneous, intralumbar, intralymphatic, intramammary, intramuscular, intranasal, intraneural, intraocular, intraosseous, intraperitoneal, intrapleural, intrapulmonary, intraruminal, intrathecal, intratracheal, intraurethral, intrauterine, intravenous, intraventricular, intravitreal, oral, peroral, parenteral, peridural, perineural, percutaneous, rectal, retrobulbar, subconjunctival, subcutaneous, sublingual, topical, transdermal, transmucosal and/or vaginal administration.

Mediating step (b) preferably comprises a modification of a target nucleic acid, e.g., by RNA interference when using a siRNA conjugate, or by inhibition of mRNA transcription when using an antisense molecule conjugate.

The present invention also provides a pharmaceutical composition comprising a conjugate as described above as the active agent together with a suitable carrier. For diagnostic or therapeutic applications, the pharmaceutical composition may be in the form of a solution, e.g. a solution for infusion or injection, a cream, ointment, tablet, suspension, powder, or the like. The composition may be administered in any suitable way, e.g. by parenteral administration, e.g. injection or infusion, by transmucosal application, or by transdermal application, or by oral, topical, nasal, rectal application, etc.

The pharmaceutical composition may comprise the conjugate as an active agent or a prodrug in any form suitable for delivery into an organism, particularly into a human organism. For example, the composition may comprise the active agent in an encapsulated form or in non-encapsulated form, together with a delivery vehicle such as a liposome and/or with a transfection reagent, or without a delivery vehicle and/or without a transfection reagent.

In certain embodiments, the conjugate of the present invention may be used in the regulation, e.g., the downregulation or the upregulation of a gene of interest in a target cell or a target organism. The gene of interest may be an endogenous gene or a gene from an exogenous pathogen, particularly a viral or bacterial gene or an endogenous disease-associated gene such as an oncogene or an autoimmune disease- or an allergic disease-associated gene.

In further embodiments, the conjugate of the present invention may be used in the introduction and optionally expression of coding, e.g. protein coding nucleic acid molecules such as mRNA molecules in a target cell or a target organism.

A specific aspect of the invention relates to the use of the conjugate for a target cell- and/or target organ-specific delivery of a nucleic acid molecule, particularly of a therapeutic nucleic acid molecule as herein described.

For example, the target cell may be a lung cell, a heart cell, a kidney cell, a liver cell, a pancreas cell, a colon cell, a muscle cell, a neural cell, a stomach cell, a small intestine cell, large intestine cell, a rectum cell, a bladder cell, a bone cell, an adrenal gland cell, a cell of the eye, a skin cells, or a brain cell.

Still a further aspect of the invention relates to an *in vitro* use of the conjugate for the delivery of a nucleic acid molecule into a target cell. The target cell may be selected from any type of cell into which a nucleic acid molecule may be delivered, e.g. animal cells such as mammalian cells, bird cells or insect cells, plant cells, fungal cells, protozoan cells, bacterial cells and archaea cells. In particular embodiments, the target cell is a human cell.

A specific embodiment of this aspect relates to the use of the conjugate in the down-regulation of a gene in a target cell.

In particular, the invention provides a target cell-specific delivery of a nucleic acid molecule *in vitro.* For example, the target cell may be a lung cell, a heart cell, a kidney cell, a liver cell, a pancreas cell, a colon cell, a muscle cell, a neural cell, a stomach cell, a small intestine cell, large intestine cell, a rectum cell, a bladder cell, a bone cell, an adrenal gland cell, a cell of the eye, a skin cells, or a brain cell.

A still further aspect of the present invention relates to the use of a 2',3'-O-ketal moiety (IV) as herein described for the attachment to a nucleic acid molecule.

Further aspects of the invention are as described in the following items of the specification.

### Items

1. A nucleic acid conjugate of the formula (I) comprising at least one 2',3'-O-ketal moiety: 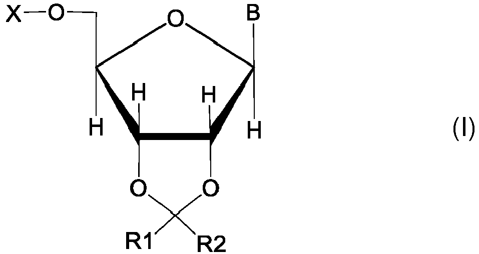 wherein
   X is a nucleic acid molecule,
   B is a nucleobase, and
   R1 and R2 are independently from each other hydrocarbon groups wherein said hydrocarbon groups optionally comprise one or more heteroatoms such as N, O, P, S, or halo.
2. The conjugate of item 1,
   wherein R1 and R2 are independently from each other C₅₋₂₁ hydrocarbon groups, particularly C₅₋₁₁ hydrocarbon groups and more particularly C₉ hydrocarbon groups wherein said hydrocarbon groups optionally comprise one or more heteroatoms such as N, O, P, S, or halo.
3. The conjugate of item 1 or 2,
   wherein R1 and R2 are independently from each other linear or branched alkyl groups, linear or branched alkenyl groups, and linear or branched alkynyl groups wherein said alkyl, alkenyl or alkynyl groups optionally comprise one or more heteroatoms such as N, O, P, S, or halo.
4. The conjugate of any one of the preceding items,
   wherein R1 and R2 are linear or branched C₉ alkyl groups.
5. The conjugate of any one of the preceding items,
   wherein the nucleobase B is a mono- or bicyclic aromatic or heteroaromatic base comprising at least one N atom.
6. The conjugate of any one of the preceding items,
   wherein the nucleobase B is a heterocyclic base, e.g. a purine or pyrimidine base, particularly selected from uracil, thymine, or an analogue thereof.
7. The conjugate of any one of the preceding items,
   wherein the nucleobase B is a modified base, e.g. a base substituted with a hydrophobic moiety, a base substituted with a carbohydrate moiety or a base substituted with a functional moiety.
8. The conjugate of item 7,
   wherein the hydrophobic moiety is a terpene moiety, particularly a C₅-C₃₀ terpene moiety, more particularly a C₅-C₂₀ terpene moiety, and even more particularly a C₁₅ terpene moiety, or a sterol moiety, e.g. a cholesterol moiety.
9. The conjugate of item 7,
   wherein the carbohydrate moiety is a GalNAc group.
10. The conjugate of item 7,
   wherein the functional moiety is a click-functional moiety such as an alkyne group or an azide group, or a such as an alkyne group or an azide group, or an ether, ester, amide, carboxylic acid, thioester, thioamide, or thioether group.
11. The conjugate of any one of items 1-6,
   wherein B is a nucleobase of the formula (IIa): 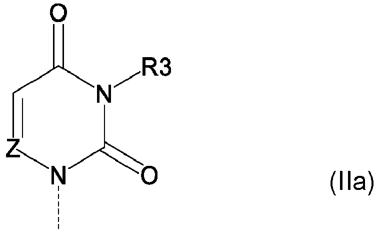 wherein Z is CH or N and R3 is H.
12. The conjugate of any one of items 1-8,
   wherein B is a nucleobase of the formula (IIb): 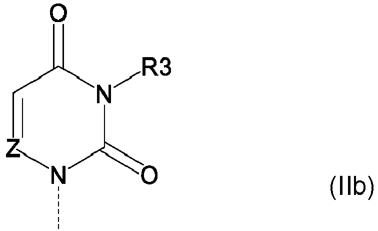 wherein Z is CH or N and R3 is a terpene moiety, particularly a C₅-C₃₀ terpene moiety, more particularly a C₅-C₂₀ terpene moiety, and even more particularly a C₁₅ terpene moiety, e.g. a terpene moiety of the formula (III): 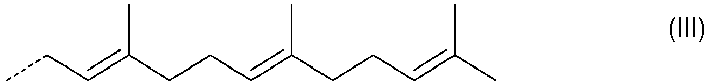
13. The conjugate of any one of the preceding items,
   wherein the nucleic acid molecule X has a strand length of at least 5 nucleotide building blocks and up to 1,000 or more nucleotide building blocks, between 5 to 100 nucleotide building blocks, between 10 and 50 nucleotide building blocks, between 12 and 40 nucleotide building blocks and particularly between 15 and 30 nucleotide building blocks.
14. The conjugate of any one of the preceding items,
   wherein the nucleic acid molecule X comprises at least one modified nucleotide building block, which is particularly selected from:
   (a) a base-modified nucleotide building block;
   (b) a sugar-modified nucleotide building block;
   (c) a backbone-modified nucleotide building block and
   (d) any combination thereof.
15. The conjugate of item 14,
   wherein the base-modified nucleotide building block is selected from a building block comprising a base substituted with a hydrophobic moiety, a nucleotide building block comprising a base substituted with a carbohydrate moiety and a nucleotide building block comprising a base substituted with a functional moiety.
16. The conjugate of item 14,
   wherein the sugar-modified nucleotide building block is selected from a 2'-modified ribose building block, a 2'-4'-bridged modified ribose building block, e.g., a locked nucleotide (LNA) building block, a morpholino building block and a peptidic nucleic acid (PNA) building block.
17. The conjugate of item 14,
   wherein the backbone-modified nucleotide building block comprises a modified internucleosidic linkage, e.g., a phosphorothioate linkage, an alkyl phosphonate, e.g., methyl phosphonate linkage, and a borano phosphate linkage.
18. The conjugate of any one of the preceding items,
   wherein the nucleic acid molecule X is a single-stranded nucleic acid molecule or a double-stranded nucleic acid molecule.
19. The conjugate of any one of items 1-18,
   wherein the nucleic acid molecule X is (i) an RNA molecule optionally comprising at least one modified nucleotide building block and/or at least one DNA building block, or (ii) a DNA molecule optionally comprising at least one modified nucleotide building block and/or at least one RNA building block, or (iii) a nucleic acid analogue molecule.
20. The conjugate of item 19,
   wherein the nucleic acid molecule X is a single-stranded, a double-stranded, a triple-stranded, or a quadruple-stranded RNA molecule, particularly a single or double stranded RNA molecule, wherein the RNA molecule optionally comprises at least one deoxyribonucleotide building block, at least one modified building block and optionally having at least one 3'-overhang, particularly a siRNA molecule.
21. The conjugate of item 19,
   wherein the nucleic acid molecule X is a, a single-stranded, a double-stranded, a triple-stranded, or a quadruple-stranded RNA molecule, particularly a single or double stranded DNA molecule, wherein the DNA molecule optionally comprises at least one ribonucleotide building block, at least one modified building block, particularly an antisense molecule.
22. The conjugate of any one of the preceding items,
   wherein the 2',3'-O-ketal building block is attached to the 5'-terminus and/or to the 3'-terminus of a strand of the nucleic acid molecule X.
23. The conjugate of item 22,
   wherein the 2',3'-O-ketal building block is attached to the 5'-terminus of the sense strand of a double-stranded siRNA molecule or to the 5'-terminus of an antisense molecule.
24. The conjugate of any one of the preceding items,
   wherein the 2',3'-O-ketal building block is attached to a base within a strand of the nucleic acid molecule X.
25. The conjugate of any one of the preceding items for use in medicine, e.g. for use in human medicine or veterinary medicine, particularly for use in human medicine.
26. The conjugate of any one of items 1-24 for use according to item 25, wherein the conjugate is administered to a subject in need thereof, particularly to a human subject.
27. The conjugate of any one of items 1-24 for use according to item 25, wherein the conjugate is administered to a target cell or a target organ *ex vivo* and wherein the target cell or target organ is subsequently introduced into a subject in need thereof, particularly into a human subject.
28. The conjugate of any one of items 1-24 for use according to item 25, wherein the conjugate is administered to an oocyte or embryo, wherein the use of human stem cells or human embryos for industrial commercial purposes is excluded.
29. The conjugate of any one of items 1-24 for use according to any one of items 25-28, wherein the administration comprises a target cell- and/or target organ-specific delivery.
30. The conjugate of any one of items 1-24 for use according to any one of items 25-29 in the upregulation or downregulation of a gene in a target cell or a target organism, e.g., an endogenous gene or a gene from an exogenous pathogen, particularly a viral or bacterial gene or an endogenous disease-associated gene such as an oncogene or an autoimmune disease- or allergic disease-associated gene.
31. The conjugate of any one of items 1-24 for use according to any one of items 25-29 in the introduction and optionally expression of a coding nucleic acid molecule gene in a target cell or a target organism.
32. The conjugate of any one of items 1-24 for use according to any one of items 25-31 wherein the target cell is a lung cell, a heart cell, a kidney cell, a liver cell, a pancreas cell, a colon cell, a muscle cell, a neural cell, a stomach cell, a small intestine cell, large intestine cell, a rectum cell, a bladder cell, a bone cell, an adrenal gland cell, a cell of the eye, a skin cells, or a brain cell.
33. *In vitro* use of the conjugate of any one of items 1-24 for the delivery of a nucleic acid molecule into a target cell.
34. The use of item 33,
   wherein the target cell is selected from animal cells such as mammalian cells, bird cells or insect cells, plant cells, fungal cells, protozoan cells, bacterial cells, and archaea cells.
35. The use of item 33 or 34,
   wherein the target cell is a human cell.
36. The use of any one of items 33-35 for the upregulation or downregulation of a gene in the target cell.
37. The use of any one of items 33-35 for the introduction and optionally expression of a coding nucleic acid molecule gene in a target cell or a target organism.
38. The use of any one of items 33-37 comprising a target cell-specific delivery.
39. The use of any one of items 33-38,
   wherein the target cell is a lung cell, a heart cell, a kidney cell, a liver cell, a pancreas cell, a colon cell, a muscle cell, a neural cell, a stomach cell, a small intestine cell, large intestine cell, a rectum cell, a bladder cell, a bone cell, an adrenal gland cell, a cell of the eye, a skin cells, or a brain cell.
40. Use of a 2',3'-O-ketal moiety (IV): 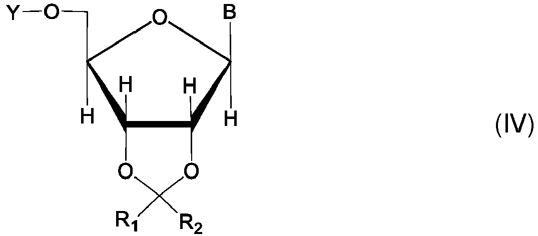 wherein
   Y is a reactive functional moiety,
   B is a nucleobase, and
   R1 and R2 are independently from each other hydrocarbon groups wherein said hydrocarbon groups optionally comprise one or more heteroatoms such as N, O, P, S or halo,
   for the attachment to a nucleic acid molecule.
41. The use of item 40,
   wherein the 2',3'-O-ketal moiety (IV) comprises at least one feature of any one of items 2-12.
42. The use of item 40 or 41,
   wherein the nucleic acid molecule comprises at least one feature of any one of items 13-21.
43. The use of any one of items 40-42,
   wherein the attachment of the 2',3'-O-ketal moiety (IV) to the nucleic acid molecule comprises at least one feature of any one of items 22-24.
44. The use of any one of items 40-43,
   wherein the reactive functional moiety Y is a phosphoroamidate group.

The present invention shall be outlined in more detail by the following Figures and Examples.

### Figure Legends:

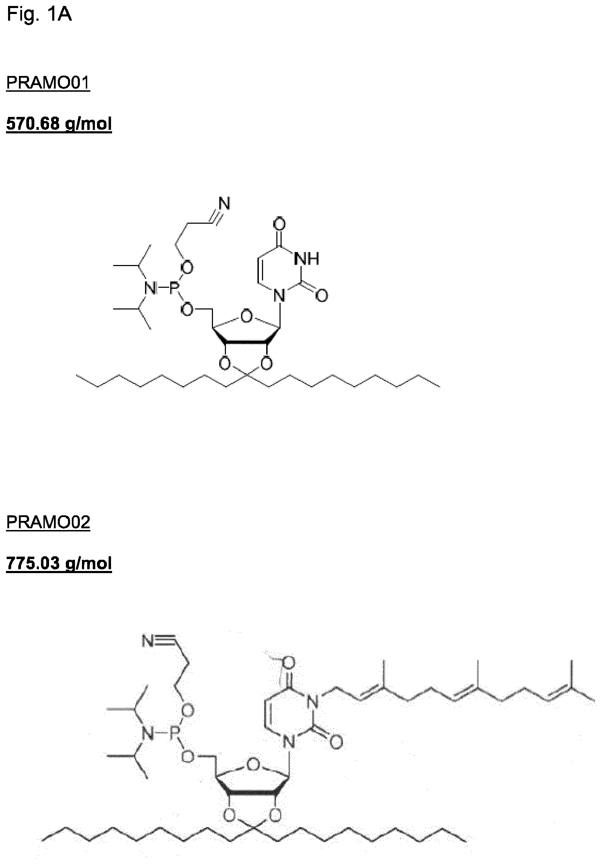

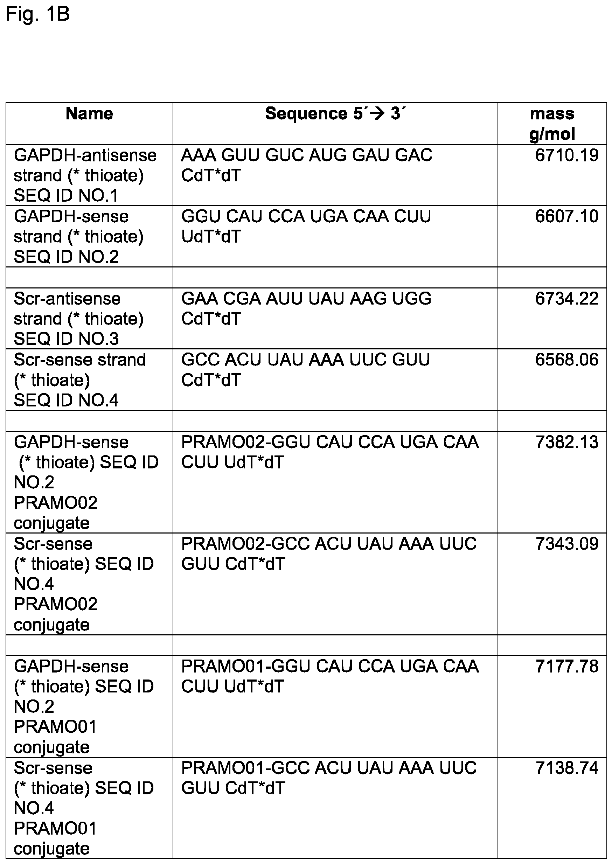

**Figure 1****: A.** Structure and molar mass of 2',3'-O-ketal moieties PRAMO01 and PRAMO02 as phosphoramidites. **B.** Sequence and molecular mass of oligonucleotides GAPDH-antisense and GAPDH-sense (together forming the targeting double-stranded siRNA molecule siGAPDH) and Scr-antisense and Scr-sense (together forming the non-targeting double-stranded siRNA molecule Scrambled siRNA) as naked RNA molecules or as conjugates with PRAMO01 or PRAMO02. * means a phosphorothioate internucleosidic linkage. dT means a 2'-deoyribonucleotide T building block.

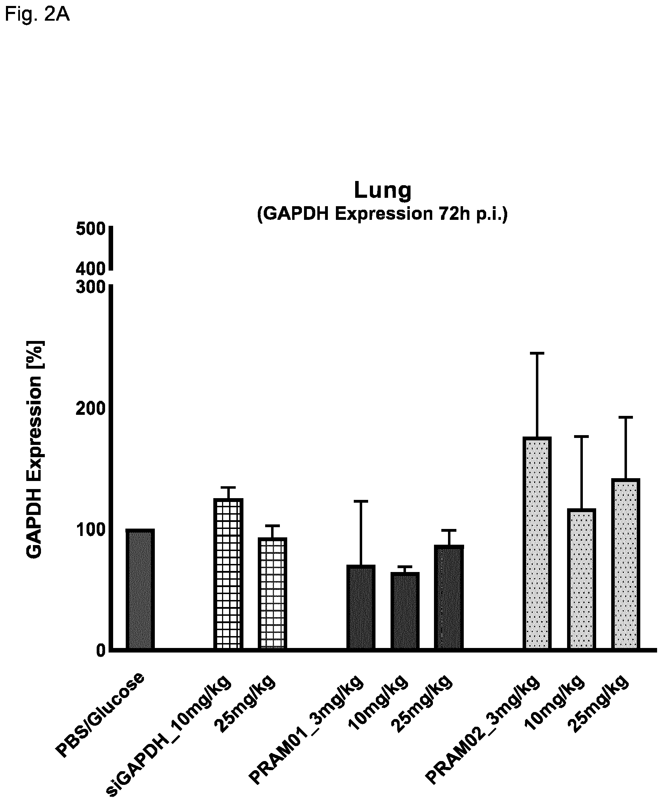

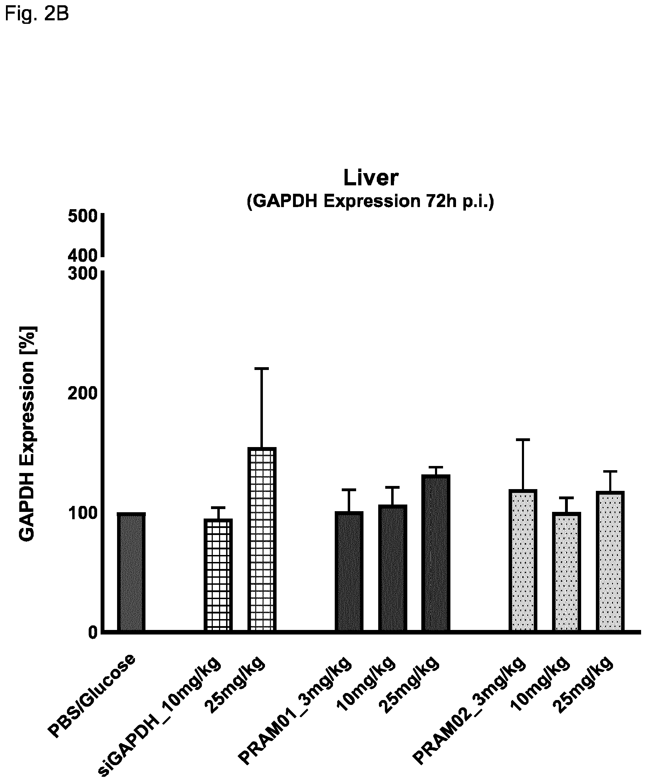

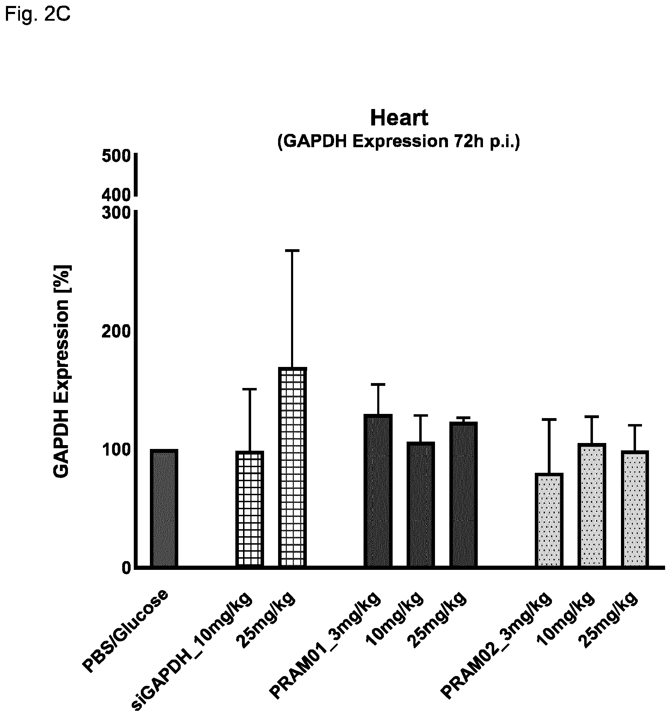

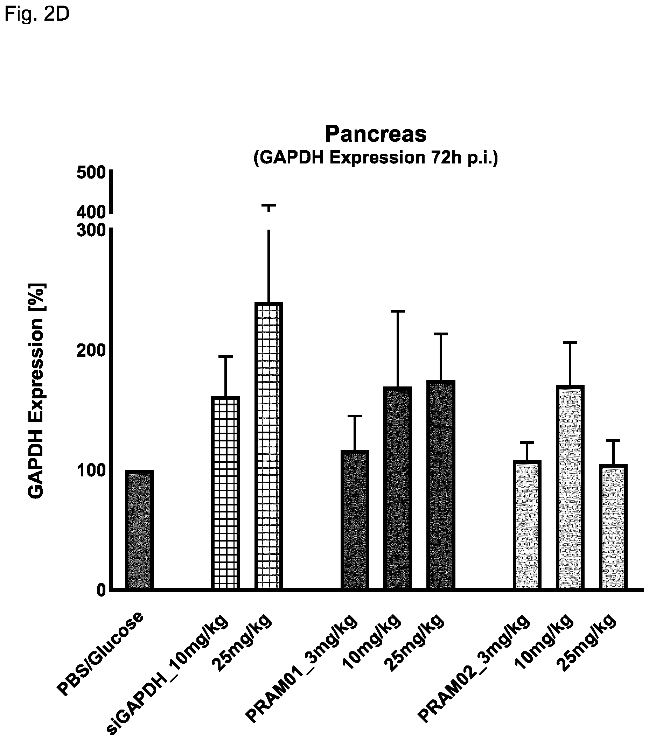

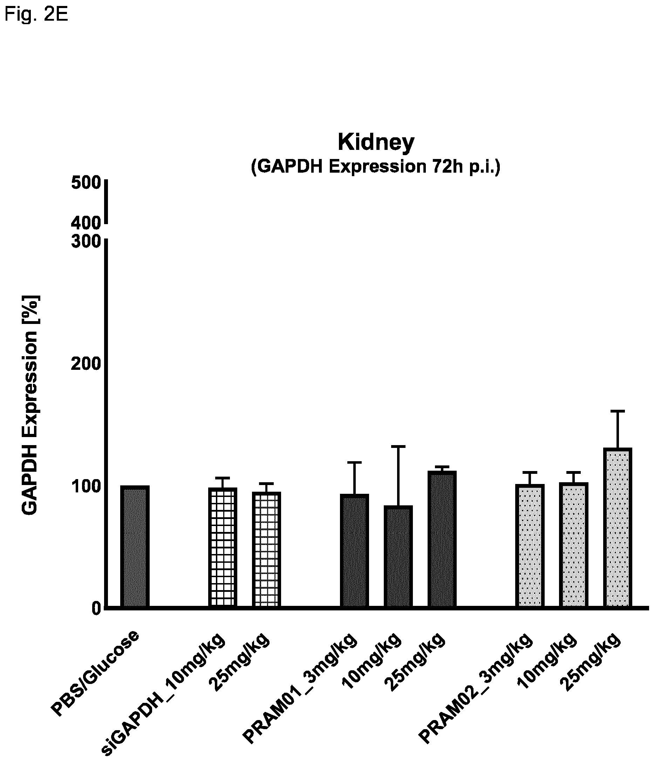

**Figure 2****:** Measurement of GAPDH mRNA levels in lung (**A**), liver (**B**), heart (**C**), pancreas (**D**) and kidney (**E**) of Balb/c mice (6-8 weeks) injected intravenously with 3, 10, or 25 mg/kg of naked siRNA (siGAPDH), PRAMO01 conjugated siRNA, PRAMO02 conjugated siRNA or PBS/glucose (n=3 per group). The tissues were collected after 72 hours post-injection. The GAPDH mRNA levels were measured using ΔΔC_{T} normalized to a housekeeping gene (Actin B), and presented as percentage of phosphate buffered saline (PBS)/glucose (mean ± SD).

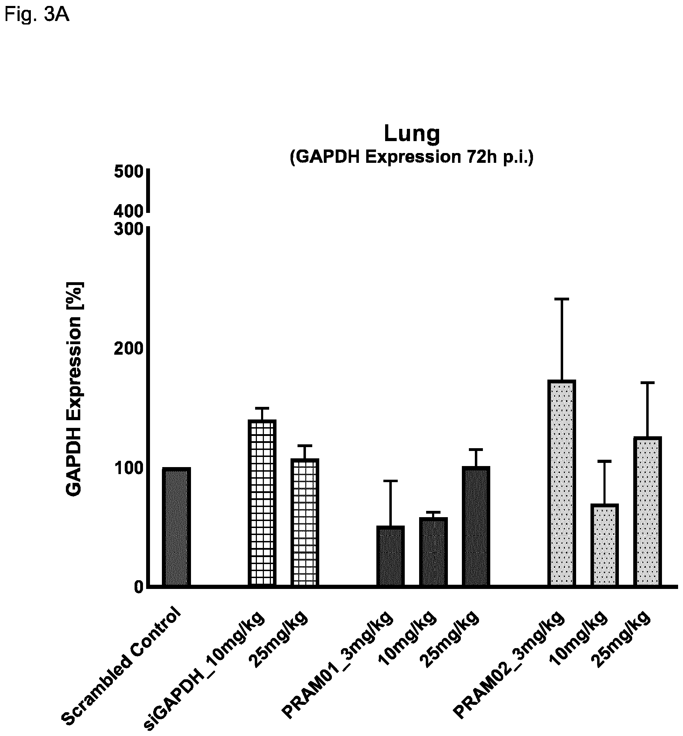

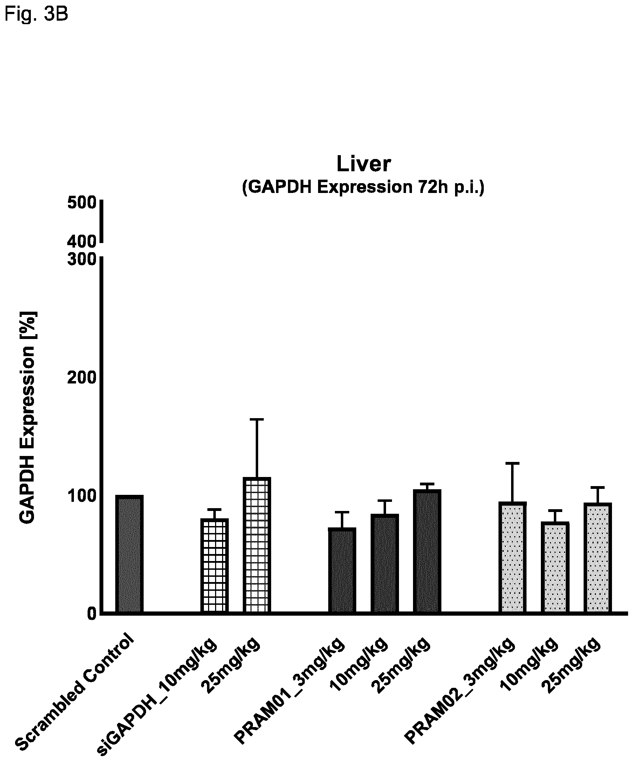

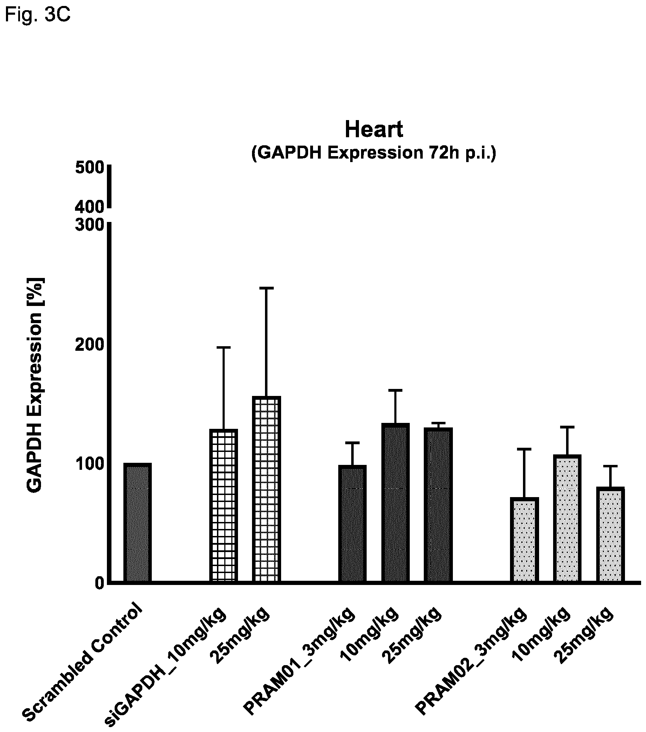

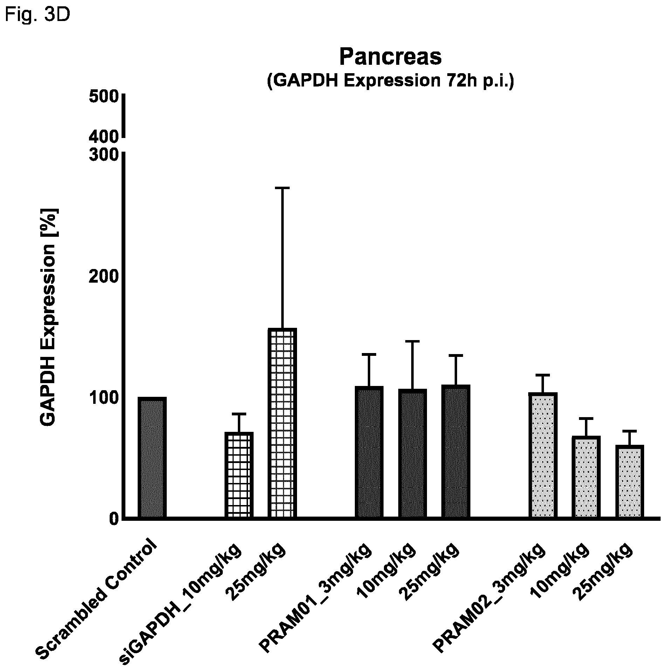

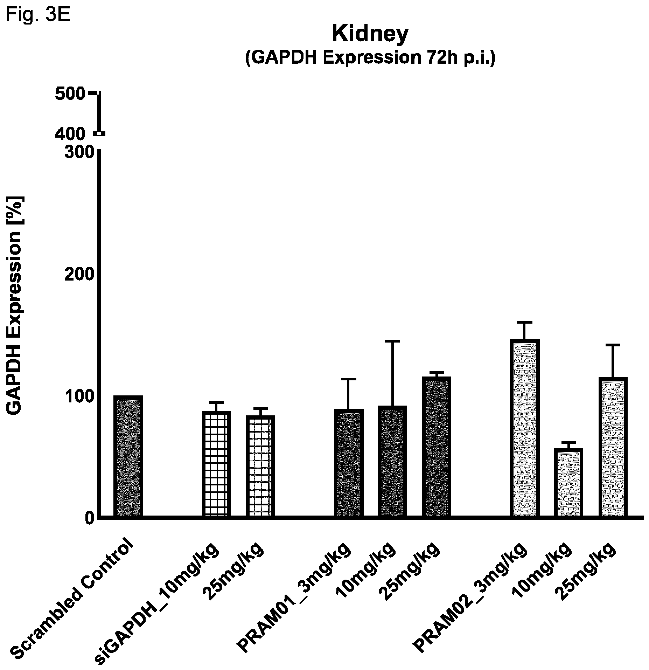

**Figure 3****:** Measurement of GAPDH mRNA levels in lung (**A**), liver (**B**), heart (**C**), pancreas (**D**) and kidney (**E**) of Balb/c mice (6-8 weeks) injected intravenously with 3, 10, or 25 mg/kg of naked siRNA (siGAPDH), PRAMO01 conjugated siRNA, PRAMO02 conjugated siRNA, non-targeting control siRNA (Scrambled siRNA) (n=3 per group). The tissues were collected after 72 hours post-injection. The GAPDH mRNA levels were measured using ΔΔC_{T} normalized to a housekeeping gene (Actin B), and presented as percentage of non-targeting siRNA (mean ± SD).

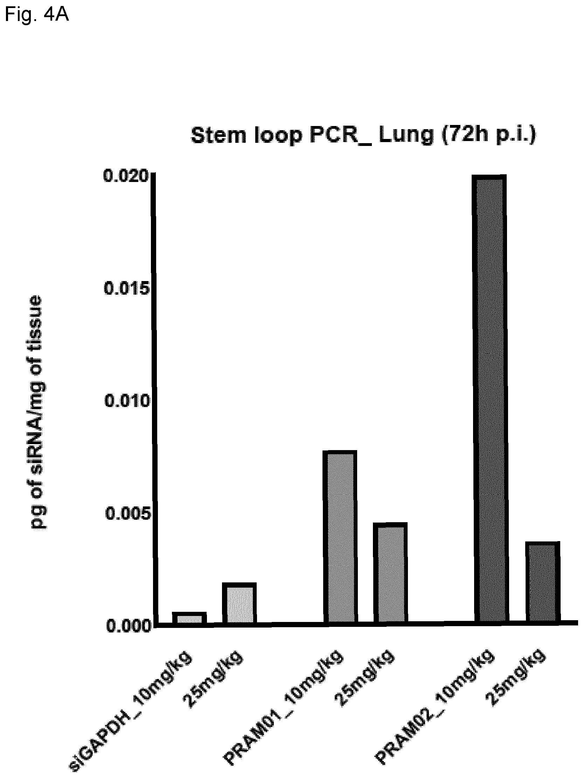

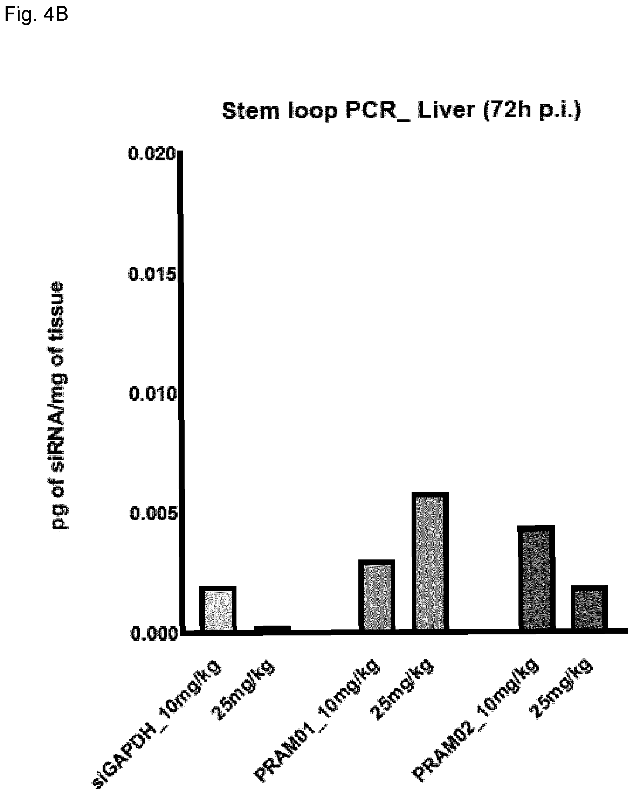

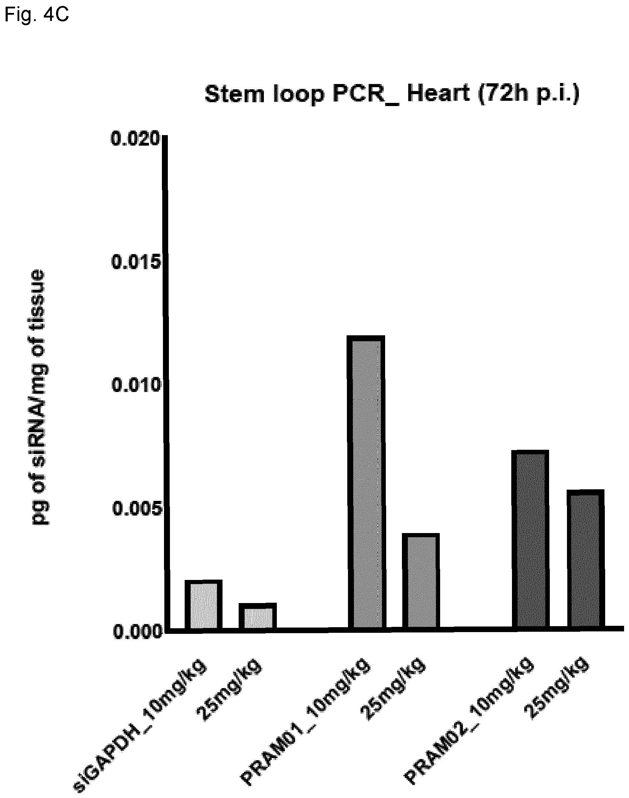

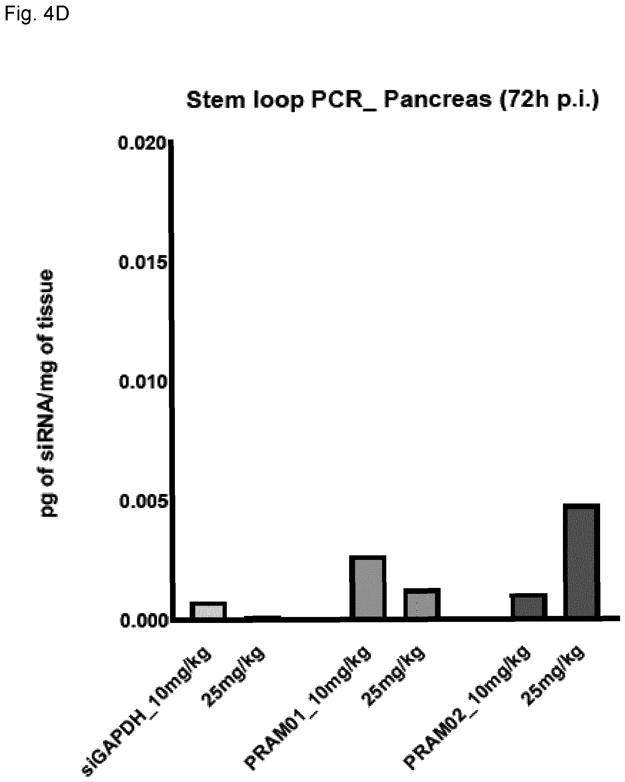

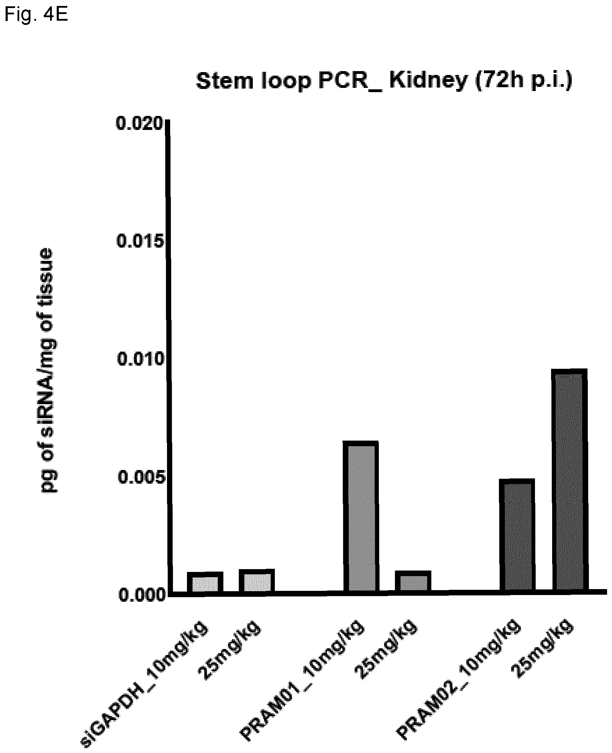

**Figure 4****:** Measurement of GAPDH siRNA concentration in lung (**A**), liver (**B**), heart (**C**), pancreas (**D**) and kidney (**E**). Balb/c mice (6-8 weeks) injected intravenously with 10 and 25 mg/kg of naked siRNA (siGAPDH), PRAMO01 conjugated siRNA, and PRAMO02 conjugated siRNA (n=3 per group). The tissues were collected after 72 hours post-injection. The GAPDH siRNA concentration was measured using stem-loop qPCR method based on standard curve (mean ± SD).

### Examples

### Results

Two different lipid-based carrier molecules PRAMO01 and PRAMO02 (Fig. 1) were attached to the sense strand of a double-stranded siRNA molecule siGAPDH directed against the glyceryl aldehyde 3-phosphate dehydrogenase (GAPDH) gene. The modified siRNA molecules ware transferred into different organs *in vivo* and the down-regulation of the GAPDH gene was analyzed.

PRAMO01 and PRAMO02 were synthetized as modified phosphoramidites and covalently coupled to the 5' end of the sense RNA strand.

Figures 2-3 illustrate the results of the intravenous injection of lipid-modified oligonucleotides into the tail vein of Balb/C mice. GAPDH gene expression in the target organs of the experimental animals was specifically altered. The animals were sacrificed 72 hours after injection, and 5 different tissues were examined. A down-regulation (gene-silencing) of the GAPDH gene to 58% was detected using PRAMO01 modified siRNAs in lung (n=3). Down-regulation could also be detected using PRAMO02 modified siRNAs in liver, heart, and pancreas (n=3).

Figure 4 illustrates the result of stem-loop qPCR in Balb/C mice, which were intravenously injected with naked siRNA, PRAMO01 modified siRNA and PRAMO02 modified siRNA in different concentrations. GAPDH concentration were measured in 5 different organs 72 hours post injection. The GAPDH siRNA could be found in all organs especially in lung and heart tissues.

### Material and Methods

### Synthesis of lipids and oligonucleotides

Lipid synthesis was performed according to standard methods. Synthesis of oligonucleotides and conjugation of oligonucleotides to lipids were performed according to standard methods.

### Injection of naked and lipid-conjugated siRNAs into mice

Animal experiments were performed in Preclinics GmbH (Potsdam, Germany) and in EPO Experimentelle Pharmakologie & Onkologie Berlin-Buch GmbH (Berlin, Germany). Female Balb/c mice 6-weeks-old were injected intravenously with glucose or 3, 10, or 25 mg/kg siRNA (naked or lipid-conjugated dissolved in injection water). In general, three mice per substance were injected (n=33, including controls). The animals were sacrificed day 3 post-injection.

### Measurement of in vivo mRNA silencing

Tissues were collected, snap-frozen and stored at -80°C. RNA extraction was performed using Qiagen TissueLyser and RNeasy Kit (Qiagen) in TRIzol RNA Isolation Reagent (ThermoFisher Scientific, Germany). Using RevertAid H Minus First Strand cDNA Synthesis Kit and random Hexamer primers (MBI Ferments, St. Leon-Rot, Germany), cDNAs were transcribed. For qPCR, TaqMan GAPDH Assay, TaqMan ACTINB Assay, and TaqMan Mastermix (ThermoFischer Scientific, Germany) were used according to the manufacturer's instructions. The relative GAPDH expression was normalized to Actin B and determined by ΔΔCt method.

### Stem-loop PCR

To amplify siRNA, the isolated RNA was transcribed into cDNA using the RevertAid H Minus First Strand cDNA Synthesis Kit (ThermoFisher Scientific, Germany). For the quantification of siRNA, a Cybergreen based qPCR assay (Biorad) was used. All procedures were performed according to the manufacturer's instructions.

### Statistical analysis

Data was analysed using GraphPad Prism 8 software (GraphPad Software, Inc., San Diego, CA, USA). In each of the three independent *in vivo* experiments, the level of silencing was normalized to the mean of the non-targeting controls (corresponding scrambled siRNA). Data was analyzed using T-test and ANOVA for multiple comparisons.

## Claims

1. A nucleic acid conjugate of the formula (I) comprising at least one 2',3'-O-ketal moiety: 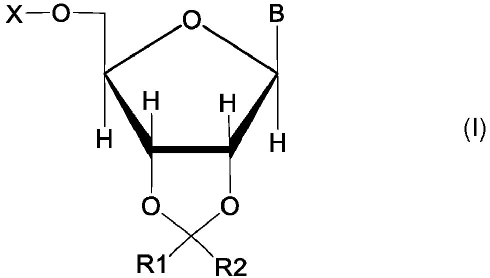 wherein
X is a nucleic acid molecule,
B is a nucleobase, and
wherein R1 and R2 are independently from each other C₅₋₂₁ hydrocarbon groups, particularly C₅₋₁₁ hydrocarbon groups and more particularly C₉ hydrocarbon groups wherein said hydrocarbon groups optionally comprise one or more heteroatoms such as N, O, P, S, or halo.

2. The conjugate of claim 1,
wherein R1 and R2 are linear or branched C₉ alkyl groups.

3. The conjugate of any one of the preceding claims,
wherein the nucleobase B is a heterocyclic base, e.g., a purine or pyrimidine base, particularly selected from uracil, thymine, or an analogue thereof, and/or
wherein the nucleobase B is a modified base, e.g., a base substituted with a hydrophobic moiety, a base substituted with a carbohydrate moiety or a base substituted with a functional moiety, wherein the hydrophobic moiety is particularly a terpene moiety, particularly a C₅-C₃₀ terpene moiety, more particularly a C₅-C₂₀ terpene moiety, and even more particularly a C₁₅ terpene moiety, or a sterol moiety, e.g., a cholesterol moiety.

4. The conjugate of any one of claims 1-3,
wherein B is a nucleobase of the formula (IIa): 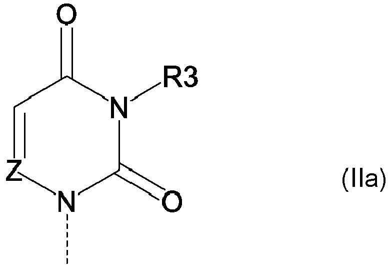 wherein Z is CH or N and R3 is H;
or wherein B is a nucleobase of the formula (IIb): 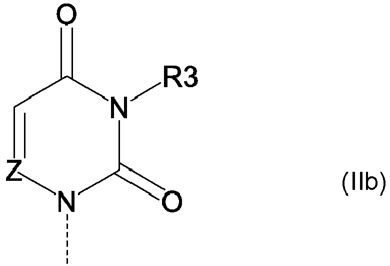 wherein Z is CH or N and R3 is a terpene moiety, particularly a C₅-C₃₀ terpene moiety, more particularly a C₅-C₂₀ terpene moiety, and even more particularly a C₁₅ terpene moiety, e.g., a terpene moiety of the formula (III):

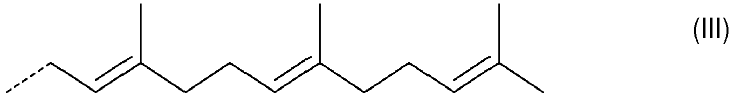

5. The conjugate of any one of the preceding claims,
wherein the nucleic acid molecule X has a strand length of at least about 2 nucleotide blocks and up to 1,000 or more nucleotide building blocks, between 5 to 100 nucleotide building blocks, between 10 and 50 nucleotide building blocks, between 12 and 40 nucleotide building blocks and particularly between 15 and 30 nucleotide building blocks.

6. The conjugate of any one of the preceding claims,
wherein the nucleic acid molecule X comprises at least one modified nucleotide building block, which is particularly selected from:
(a) a nucleobase-modified nucleotide building block;
(b) a sugar-modified nucleotide building block;
(c) a backbone-modified nucleotide building block and
(d) any combination thereof.

7. The conjugate of any one of claims 1-6,
wherein the nucleic acid molecule X is (i) an RNA molecule optionally comprising at least one deoxyribonucleotide building block and/or at least one modified nucleotide building block, or (ii) a DNA molecule optionally comprising at least one ribonucleotide building block and/or at least one modified nucleotide building block, or (iii) a nucleic acid analogue molecule.

8. The conjugate of claim 7,
wherein the nucleic acid molecule X is (i) a double-stranded RNA molecule optionally comprising at least one deoxyribonucleotide building block and/or at least one modified building block and optionally having at least one 3'-overhang, particularly a siRNA molecule, or wherein the nucleic acid molecule X is (ii) a single-stranded DNA molecule optionally comprising at least one ribonucleotide building block and/or at least one modified building block, particularly an antisense molecule.

9. The conjugate of any one of the preceding claims,
wherein the 2',3'-O-ketal building block is attached to the 5'-terminus and/or to the 3'-terminus of a strand of the nucleic acid molecule X, and/or
wherein the 2',3'-O-ketal building block is attached to a base within a strand of the nucleic acid molecule X, and
wherein the 2',3'-O-ketal building block particularly is attached to the 5'-terminus of the sense strand of a double-stranded siRNA molecule or to the 5'-terminus of an antisense molecule.

10. The conjugate of any one of the preceding claims for use in medicine, e.g. for use in human medicine or veterinary medicine, particularly for use in human medicine.

11. The conjugate of any one of claims 1-9 for use according to claim 10,
wherein the conjugate is administered to a subject in need thereof, particularly to a human subject, or
wherein the conjugate is administered to a target cell or a target organ *ex vivo* and wherein the target cell or target organ is subsequently introduced into a subject in need thereof, particularly into a human subject, or
wherein the conjugate is administered to an oocyte or embryo, wherein the use of human stem cells or human embryos for industrial commercial purposes is excluded.

12. The conjugate of any one of claims 1-9 for use according to any one of claims 10-11, wherein the administration comprises a target cell- and/or target organ-specific delivery,
wherein the target cell particularly is a lung cell, a heart cell, a kidney cell, a liver cell, a pancreas cell, a colon cell, a muscle cell, a neural cell, a stomach cell, a small intestine cell, a large intestine cell, a rectum cell, a bladder cell, a bone cell, an adrenal gland cell, a cell of the eye, a skin cell, or a brain cell.

13. *In vitro* use of the conjugate of any one of claims 1-9 for the delivery of a nucleic acid molecule into a target cell.

14. The use of claim 13 comprising a target cell-specific delivery, wherein the target cell particularly is a lung cell, a heart cell, a kidney cell, a liver cell, a pancreas cell, a colon cell, a muscle cell, a neural cell, a stomach cell, a small intestine cell, a large intestine cell, a rectum cell, a bladder cell, a bone cell, an adrenal gland cell, a cell of the eye, a skin cell, or a brain cell.

15. Use of a 2',3'-O-ketal moiety (IV): 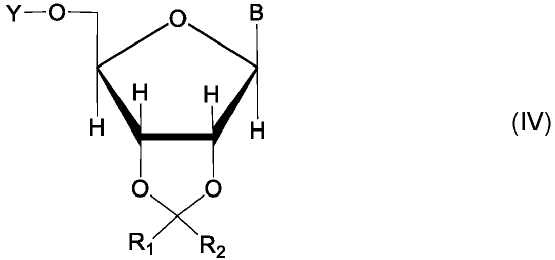 wherein
Y is a reactive functional moiety, e.g. a phosphoroamidate group,
B is a nucleobase, and
R1 and R2 are independently from each other hydrocarbon groups wherein said hydrocarbon groups optionally comprise one or more heteroatoms such as N, O, P, S, or halo,
for the attachment to a nucleic acid molecule.
